(19) Europäisches Patentamt
European Patent Office
Office européen des brevets

(11) EP 0 755 518 B1

(12) **FASCICULE DE BREVET EUROPEEN**

(45) Date de publication et mention de la délivrance du brevet:
**14.10.1998 Bulletin 1998/42**

(21) Numéro de dépôt: **95916732.1**

(22) Date de dépôt: **10.04.1995**

(51) Int Cl.[6]: **G01N 33/86**, C07K 16/36

(86) Numéro de dépôt international:
**PCT/FR95/00456**

(87) Numéro de publication internationale:
**WO 95/28644 (26.10.1995 Gazette 1995/46)**

(54) **PROCEDE D'IMMUNODOSAGE DE L'ANTITHROMBINE III ACTIVEE PAR UN GLYCOSAMINOGLYCANE, ANTICORPS MONOCLONAUX CORRESPONDANTS ET LEUR PROCEDE D'OBTENTION**

IMMUNOLOGISCHES VERFAHREN ZUM NACHWEIS VON GLYKOSAMINOGLYKAN-AKTIVIERTEM ANTITHROMBIN III; DIE ENTSPRECHENDEN MONOKLONALEN ANTIKÖRPER UND DEREN HERSTELLUNG

METHOD FOR THE IMMUNO-ASSAY OF ANTITHROMBIN III ACTIVATED BY A GLYCOSAMINOGLYCAN, CORRESPONDING MONOCLONAL ANTIBODIES AND METHOD FOR PRODUCING THEM

(84) Etats contractants désignés:
**AT BE CH DE DK ES FR GB GR IE IT LI LU MC NL PT SE**

(30) Priorité: **14.04.1994 FR 9404469**

(43) Date de publication de la demande:
**29.01.1997 Bulletin 1997/05**

(73) Titulaire: **PASTEUR SANOFI DIAGNOSTICS
92430 Marnes La Coquette (FR)**

(72) Inventeurs:
- **LORMEAU, Jean-Claude
F-94240 Kremlin-Bicètre (FR)**
- **MILLE, Blandine, Georgette, Renée
F-66000 Perpignan (FR)**
- **PAOLUCCI, Francis, Egiste, Joseph
F-34090 Montpellier (FR)**
- **PAU, Bernard
F-34080 Montpellier (FR)**

(74) Mandataire: **Le Guen, Gérard et al
CABINET LAVOIX
2, place d'Estienne d'Orves
75441 Paris Cédex 09 (FR)**

(56) Documents cités:
**EP-A- 0 165 134**
**EP-A- 0 341 927**
**DE-A- 2 708 985**
**DE-A- 3 804 590**
**FR-A- 2 645 647**

- **BIOCHEMISTRY, vol. 33, no. 14, 12 Avril 1994 EASTON US, pages 4375-4383, J. DAWES ET AL. cité dans la demande**
- **EUROPEAN JOURNAL OF BIOCHEMISTRY, vol. 180, no. 2, Mars 1989 BERLIN DE, pages 319-326, S. KNOLLER ET AL. cité dans la demande**
- **DATABASE WPI Week 9419 Derwent Publications Ltd., London, GB; AN 94-153810 & JP-A-06 094 713 (M FUNAYAMA) , 8 Avril 1994 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 755 518 B1

Printed by Jouve, 75001 PARIS (FR)

## Description

La présente invention concerne un procédé immunologique de dosage de l'antithrombine III (AT III) activée par l'un des glycosaminoglycanes sulfatés, connus pour agir sur le processus de la coagulation sanguine au niveau de l'AT III. En présence d'héparine l'AT III exerce son activité anticoagulante en inhibant différentes protéases sériques, impliquées dans les réactions conduisant à la formation de la fibrine insoluble, particulièrement la thrombine et le facteur Xa.

On connaît divers glycosaminoglycanes, analogues biochimiques, qui en se fixant sur l'AT III interrompent la suite de réactions enzymatiques aboutissant au caillot ; on peut citer notamment les héparines naturelles, de porc ou de boeuf, les héparines de bas poids moléculaire, dont plusieurs types sont commercialisés. On connaît aussi certains oligosaccharides sulfatés ayant les mêmes propriétés, dont ceux décrits dans les demandes de brevet EP-A-84 999, EP-A-133 599 et EP-A-356 275, notamment le pentasaccharide reconnu comme constituant la séquence des chaînes d'héparine sur lequel l'AT III se fixe.

Ces composés peuvent être utilisés chez l'homme comme anticoagulants et antithrombotiques dans des traitements prophylactiques ou thérapeutiques ; toutefois, le suivi de l'état du système de la coagulation au cours de ces traitements est indispensable étant donné leurs effets secondaires potentiels et notamment leurs risques hémorragiques, variables selon les individus.

La surveillance repose actuellement sur des méthodes biologiques qui consistent à déterminer in vitro l'action inhibitrice au niveau du facteur Xa, des glycosaminoglycanes sulfatés présents dans le sang du patient, soit par un test de coagulation spécifique de ce facteur, soit par la mesure de son activité résiduelle sur un substrat chromogène. On peut pour la description de ces tests, se référer à l'ouvrage : l'héparine - J.P. Duclos - Editions Masson (1984).

Chez l'homme, les concentrations sanguines efficaces des glycosaminoglycanes antithrombotiques sont faibles, inférieures à 10 µM et en l'absence d'anticorps spécifiques, aucun dosage direct de ces produits, utilisable en routine dans les laboratoires d'analyses biologiques, n'a été développé.

Pourtant, il faut noter que les résultats obtenus avec les deux méthodes précédentes sont parfois divergents et mal corrélés avec l'activité antithrombotique observée in vivo et avec l'importance des risques hémorragiques comme le mentionnent A. Leizorovicz, L. Bara, M.M. Samama et M.C. Haugh dans Haemostasis (1993), 23 (sup. 1), p. 89-98. En outre, ils ne reflètent pas toujours la situation clinique et notamment sous-estiment les taux élevés d'héparine et de glycosaminoglycanes analogues.

Il était donc souhaitable de disposer d'une méthode simple et fiable permettant dans la cascade de la coagulation, d'apprécier l'importance de l'action de l'un de ces glycosaminoglycanes sulfatés sur l'AT III plasmatique. On sait que cette action, au cours de laquelle l'héparine ou ses analogues se fixent transitoirement sur l'AT III, se traduit notamment par une modification conformationnelle de la protéine qui est à l'origine de l'augmentation de la vitesse d'inhibition du facteur Xa.

La détermination de la proportion, vraisemblablement faible, d'AT III dans cet état activé, c'est à dire dans une forme modifiée par la fixation de l'héparine ou de glycosaminoglycanes, qui précède la fixation covalente de l'AT III aux autres protéases de la coagulation, doit permettre de mieux apprécier, que par les méthodes biologiques antérieures, l'intensité de l'action inhibitrice du glycosaminoglycane sulfaté présent dans le plasma.

On sait que les anticorps monoclonaux sont bien adaptés à l'identification et la détermination de conformations spatiales spécifiques.

La demande JP 6094713 décrit un procédé de dosage de l'activité AT III par chromatographie d'affinité. Selon ce procédé la détermination des complexes héparine-AT III ne met en oeuvre qu'un seul type d'anticorps marqué spécifique de ces complexes. Par ailleurs, la demande DE-2 708 985 décrit un procédé d'évaluation des taux plasmatiques de l'héparine selon lequel la quantité d'AT III complexée à l'héparine est mesurée à l'aide des anticorps spécifiques du complexe AT III-thombine. Un procédé de dosage immunologique des complexes AT III-protéase, mettant en oeuvre l'utilisation d'anticorps ou de fragments d'anticorps fixant spécifiquement l'AT III complexée à une sérine estérase mais ne réagissant pas avec l'AT III libre, est décrit dans la demande FR-2 645 647. Des sondes de diagnostic pour le dosage d'AT III modifiée par l'elastase sont décrits sans la demande DE-3804590 Ce document fait état d'anticorps capables de reconnaitre l'AT III dénaturée et non l'AT III native

La présente invention concerne un procédé d'immunodosage, de type sandwich, encore appelé de type extraction-saturation, pour la détermination de l'antithrombine III activée par un glycosaminoglycane sulfaté, mettant en jeu un couple d'anticorps monoclonaux qui, associés, reconnaissent plus efficacement l'AT III activée par l'un de ces glycosaminoglycanes sulfatés que l'AT III native ou l'AT III dénaturée. L'un de ces anticorps reconnaît en solution, moins efficacement l'AT III native que l'AT III activée par l'un des glycosaminoglycanes sulfatés ; l'autre ne reconnaît en solution pratiquement pas l'AT III native ou activée mais reconnaît l'AT III dénaturée.

La présente invention a donc pour objet un procédé de dosage de l'AT III activée par un glycosaminoglycane sulfaté ou un oligosaccharide sulfaté dont l'effet in vivo sur l'AT III est analogue à celui de l'héparine, par un immunodosage de type sandwich en phase solide, caractérisé en ce que l'on détermine à l'aide d'un anticorps spécifique d'un

épitope exclusivement présent sur l'AT III dénaturée, les complexes immunologiques formés entre l'AT III activée et un anticorps spécifique de l'AT III activée.

Par AT III native, on entend l'AT III purifiée ou non, en solution dans un tampon ou dans un plasma, éventuellement dilué.

Par AT III activée par un glycosaminoglycane sulfaté antithrombotique, on entend l'AT III en solution, dans un tampon ou dans un plasma éventuellement dilué, dans lequel se trouve aussi le glycosaminoglycane, à des concentrations relatives compatibles avec leurs activités biologiques. Normalement, la concentration plasmatique de l'AT III est voisine de 2,7 µM ; elle est inférieure dans certains états pathologiques héréditaires ou acquis. Les concentrations plasmatiques maximales tolérables des glycosaminoglycanes sulfatés antithrombotiques sont fonction de leurs activités intrinsèques et de leur toxicité.

On sait par exemple, que pour l'héparine, la concentration au pic plasmatique est environ de 0,4 µM, tandis que pour les héparines de bas poids moléculaire, elle peut atteindre 2µM; pour le pentasaccharide A de formule :

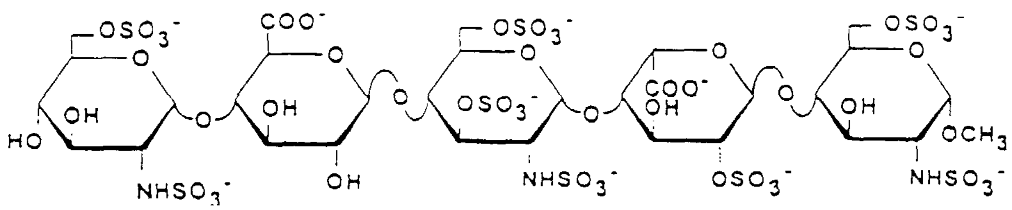

décrit dans Thromb. Res. (1986). 43, p 243-248, la concentration sera de l'ordre de 1µM.

Par AT III dénaturée, on entend l'AT III traitée par la guanidine ou un de ses sels comme décrit par J Watton et coll dans Biochem (1993), 32, p. 6501-6505, de telle sorte que sa conformation au niveau du site de liaison de l'AT III à l'héparine et de celui à la protéase qu'elle inhibe, soient modifiées.

Un autre exemple d'AT III dénaturée est l'AT III adsorbée sur la surface d'un polymère, par exemple sur celle des puits d'une plaque de microtitration ou de microbilles, en polystyrène ou en chlorure de polyvinyle, couramment utilisés en immunodiagnostic.

Dans tout le texte par AT III, on entend l'antithrombine III humaine ou de l'espèce animale chez qui on pratiquera l'immunodosage selon l'invention.

Les anticorps monoclonaux nécessaires à la mise en oeuvre du procédé d'immunodosage sont aussi un objet de l'invention.

Des anticorps monoclonaux dirigés contre des épitopes de l'antithrombine III ont déjà été décrits notamment dans Eur. J. Biochem, (1989), 180, p. 319-326, Blood Coagulation Fibrinolysis, (1990), 1, p. 619-626, ; ou Hybridoma, (1991), 10 p. 633-640. Leur fixation au complexe covalent antithrombine III/thrombine ou à l'antithrombine III en présence d'héparine, a parfois été étudiée mais il n'a pas encore été mentionné, ni suggéré, que des anticorps monoclonaux puissent se lier à l'AT III activée par la fixation d'héparine, ou de glycosaminoglycanes sulfatés analogues, mieux qu'à l'AT III native.

L'obtention d'un anticorps monoclonal spécifique de l'AT III complexée à l'héparine utile pour l'étude des changements conformationnels de l'AT III en relation avec son effet inhibiteur de la thrombine est aussi décrit dans Biochemistry (1994), 33(14), p. 4375-4383.

Cet anticorps a été préparé à partir d'un complexe covalent AT-héparine et ne fait pas de différence entre l'AT III native et l'AT III activée par un glycosaminoglycane sulfaté tel que l'héparine.

Selon un premier aspect, l'invention conceme des anticorps monoclonaux spécifiques de l'AT III activée, c'est-à-dire les anticorps monoclonaux qui reconnaissent l'antithrombine III en solution, activée par la fixation d'un glycosaminoglycane sulfaté biochimiquement analogue à l'héparine, et qui reconnaissent éventuellement l'antithrombine III en solution dans son état natif, purifiée ou non mais qui alors se fixent préférentiellement sur l'AT III activée.

La capacité des anticorps monoclonaux de l'invention à reconnaître préférentiellement l'AT III activée peut être étudiée par tout moyen permettant la mesure ou l'appréciation de l'affinité de l'anticorps pour son antigène. On peut notamment utiliser un appareil qui permet le suivi de la variation de la quantité de macromolécules, telles que des protéines, qui sont fixées, directement ou non, sur une surface de verre recouverte d'une fine couche d'or, par une mesure de l'angle de réflexion totale d'un rayon lumineux monochromatique polarisé sur ladite surface, phénomène connu sous le nom de résonance plasmonique de surface ; cette technique est décrite, notamment, dans J. Chromat., (1992), 597, p. 397-410, et EP-A-341 927.

Un appareil de ce type est commercialisé par la société Pharmacia, Suède sous la marque BIACORE® ou par Fisons Applied Sensor technology, Grande-Bretagne sous le nom lasys®. C'est l'appareil BIACOR® qui a été été utilisé par la suite.

Pour déterminer l'affinité d'un antigène pour un anticorps, l'un des deux éléments de la paire immunologique est lié sur la surface du biocapteur, tandis que l'autre se trouve dans une solution qui circule en continu au contact de la dite surface, dans la cellule de mesure ; le signal est une fonction de la quantité de matière se fixant sur le biocapteur ce qui permet la détermination de la vitesse de complexation de l'antigène et de l'anticorps. En général, l'élément "lié" à la surface, l'est par l'intermédiaire d'un anticorps qui y est fixé par une liaison covalente.

Dans la mesure où le procédé immunologique selon l'invention vise à étudier l'AT III en présence de glycosaminoglycane sulfaté en solution, c'est l'anticorps à étudier qui sera fixé sur la surface sensible, par exemple comme préconisé par le fabricant, par l'intermédiaire d'un anticorps anti-immunoglobulines de l'espèce de l'anticorps étudié tandis que l'antigène sera introduit dans la solution circulante, qui pourra être le plasma d'un sujet normal, éventuellement dilué ou une solution d'un tampon ; la concentration de l'antigène y sera fonction des caractéristiques spécifiques de l'appareil et notamment de la quantité d'anticorps lié à la surface sensible Dans les essais en présence d'héparine et des glycosaminoglycanes analogues, le rapport des concentrations de ceux-ci à l'AT III sera de l'ordre de 1 à 100 fois le rapport maximum que l'on pourra trouver in vivo ; on sait en effet que l'AT III activée est en présence d'une proportion d'AT III restée libre et il importe de déplacer l'équilibre vers l'AT III activée en utilisant un net excès d'activateur héparinique, afin de pouvoir faire une mesure significative avec cet appareil BIACORE®.

On peut aussi déterminer les affinités relatives d'un anticorps monoclonal pour l'AT III et l'AT III activée en le comparant dans le procédé de l'invention avec un anticorps monoclonal connu répondant à l'invention.

Selon un second aspect, l'invention concerne les anticorps monoclonaux qui ne reconnaissent pratiquement pas l'AT III native et l'AT III activée par les glycosaminoglycanes biologiquement analogues à l'héparine mais qui reconnaissent l'AT III dénaturée. Plus précisément, les anticorps de ce second groupe, au sens de la présente invention, sont capables de reconnaître spécifiquement les complexes immunologiques formés entre l'AT III activée et un anticorps spécifique de cette forme, tel que défini précédemment. L'absence d'affinité de ce second type d'anticorps monoclonaux vis à vis de l'AT III native ou activée peut aussi être étudiée avec un appareil analogue à celui précédemment cité, étant entendu qu'on considérera que l'anticorps ne reconnaît pratiquement pas l'AT III activée ou native quand le signal mesuré avec le BIACORE® sera plus de deux ou trois fois inférieur à celui mesuré avec les autres types d'anticorps.

L'affinité pour l'AT III dénaturée, notamment par traitement par du chlorure de guanidine, peut aussi être étudiée avec un appareil de détermination des affinités immunologiques mais aussi, plus simplement, par un immunoessai classique, par exemple en phase solide dans lequel l'AT III sera immobilisée par adsorption et l'anticorps monoclonal à étudier qui serait immunofixé sur l'AT III adsorbée après incubation, sera révélé, par exemple, par un anticorps anti-immunoglobulines de souris marqué de façon classique ; de préférence l'anticorps à étudier sera immobilisé et l'AT III dénaturée, qui y serait immunofixée après incubation, sera révélée par un anticorps polyclonal anti-AT III marqué ; dans les mêmes conditions, l'AT III native ne devra pratiquement pas être reconnue.

Parmi les anticorps monoclonaux de l'invention ne reconnaissant ni l'AT III activée, ni l'AT III native mais reconnaissant l'AT III dénaturée, on préfère particulièrement l'anticorps sécrété par l'hybridome déposé le 18 mars 1994, conformément au Traité de Budapest, auprès de la Collection allemande de microorganismes DSM, sous le numéro ACC 2167. Cet hybridome, comme ceux qui sécrètent l'un des deux types d'anticorps monoclonaux mis en oeuvre dans le procédé défini précédemment, constitue un autre aspect de l'invention.

Selon un autre de ses aspects, l'invention concerne le procédé d'obtention de ceux-ci.

Ce procédé consiste à effectuer une hybridation lymphocytaire en immunisant des animaux avec de l'antithrombine III humaine, activée par un oligosaccharide sulfaté antithrombotique puis à sélectionner les hybridomes producteurs d'anticorps monoclonaux présentant les propriétés immunologiques recherchées c'est-à-dire ceux qui ont plus d'affinité pour l'AT III activée en solution que pour l'AT III native et ceux qui reconnaissent l'AT III dénaturée par exemple par la guanidine et ses sels, notamment le chlorhydrate de guanidine et ne reconnaissent pratiquement pas l'AT III native et l'AT III activée.

Les animaux, de préférence des souris, sont immunisés par une méthode classique, par injection répétée, notamment par voie intrapéritonéale, d'AT III humaine puis de l'oligosaccharide choisi ou du mélange d'AT III et d'oligosaccharide. Il n'est en général pas nécessaire de répéter l'injection de l'AT III tandis qu'une injection journalière d'oligosaccharide, devra être pratiquée, pendant 4 à 6 jours, pour maintenir l'AT III en présence d'une forte concentration de l'oligosaccharide pendant un certain temps. L'homme du métier adaptera le nombre d'injections à la durée de vie de l'activateur. Pour l'immunisation les oligosaccharides sulfatés convenant à l'activation de l'AT III seront choisis parmi les molecules de 5 à 20 unités saccharidiques, de préférence de 5 a 10, qui sont biologiquement équivalentes à l'héparine et activent l'AT III, telles que celles décrites dans les brevets européens EP-84 999, EP-133 599 et EP-356 275 qui peuvent être préparées par synthèse chimique.

On préfère particulièrement le pentasaccharide A précédemment cité.

Les doses d'AT III injectées doivent être suffisantes pour déclencher une réaction immunitaire, y compris contre la forme activée, mais ne doivent pas être à l'origine d'hémorragies graves ; en général, on injectera de 20 µg à 60 µg d'AT III pour une souris de 25 g environ.

Si un anticorps monoclonal de l'héparine a été décrit récemment dans Anal. Biochem, (1992), 201, p. 1-8, qui ne reconnaît pas un épitope lié à l'activité anticoagulante, les oligosaccharides ne sont en général pas immunogènes par eux-mêmes, de telle sorte qu'on peut les administrer à des doses permettant d'obtenir des concentrations suffisantes pour qu'une réaction immunologique sensible se produise contre l'AT III activée, qui s'est formée chez l'animal. Il est évident que la dose journalière d'oligosaccharide injectée doit aussi être compatible avec la survie des souris ; pour le pentasaccharide A, elle peut être comprise entre 10 et 30 µg par souris.

Pour les oligosaccharides de durée de vie plus longue, une seule injection du mélange est en général suffisante.

Les injections peuvent avantageusement être répétées après quelques jours ou quelques semaines. Le protocole d'immunisation n'est pas critique, du moment que l'on tient compte des risques de toxicité, notamment des hémorragies possibles. On peut notamment appliquer la méthode décrite dans Eur. J. Biochem, (1989), 180, p. 319-326.

On sélectionne alors les souris pouvant convenir à l'hybridation lymphocytaire en recherchant l'immunosérum qui présente la plus forte réaction immunologique vis-à-vis de l'AT III en solution ; on a en effet constaté que dans un tel sérum polyclonal on trouve à la fois des anticorps dirigé contre l'AT III native et l'AT III activée en solution et contre l'AT III dénaturée et il n'est pas nécessaire à ce stade de mettre en oeuvre des procédés de sélection plus complexes. L'affinité du sérum pour l'AT III en solution est déterminée par une méthode immunométrique classique, en immobilisant le sérum à étudier par adsorption sur une surface de plastique ou par l'intermédiaire d'un anticorps anti-IgG de souris, avant une incubation avec une solution d'AT III native, puis après lavage, en mettant en contact un anticorps anti-AT III marqué, suivi d'une étape classique de révélation.

L'isolement des splénocytes et l'hybridation cellulaire sont ensuite effectués selon un procédé connu ; on pourra notamment se référer à l'article de J. Immunol. Methods, (1980), 35, p. 1-21, "Production of monoclonal antibodies : strategy and tactics", ou aux publications décrivant la préparation d'anticorps anti- AT III, et par exemple à Biochem. Biophys. Acta, (1988), 952, p. 37-47.

L'identification des hybridomes sécrétant des anticorps monoclonaux qui présentent l'une ou l'autre des affinités caractéristiques des différents types d'anticorps conformes à l'invention, peut être réalisée à l'aide d'un appareil permettant de déterminer l'intensité des réactions immunologiques, tel que ceux précédemment cités.

On peut aussi, lorsque l'on a précédemment isolé un anticorps répondant à l'invention, rechercher un anticorps complémentaire en mettant en oeuvre un immunoessai de type sandwich, avec comme antigènes selon le cas AT III native, activée ou dénaturée.

Par anticorps complémentaires, on entend un anticorps de l'invention qui reconnaît mieux l'AT III activée que l'AT III native et un anticorps de l'invention qui reconnaît l'AT III dénaturée et pratiquement pas l'AT III native et activée, de telle sorte que mis en oeuvre dans un procédé de dosage immunométrique de type sandwich par exemple, ils reconnaissent préférentiellement l'AT III activée en solution, sachant qu'elle est toujours en présence d'AT III native.

En effet, il a été mis en évidence que l'anticorps complémentaire (à l'anticorps de l'invention qui reconnaît mieux l'AT activiée que l'AT native) reconnaît l'AT III dénaturée et pratiquement pas l'AT III native et activée. Ceci permet de sélectionner des anticorps utiles à l'invention. En effet, cette spécificité correspond à la capacité de reconnaître l'AT soluble complexée avec un anticorps de l'invention. Plus précisemmment, la liaison de l'AT III avec l'anticorps spécifique de la forme activée, favorise l'apparition d'un épitope qui se trouve être également et exclusivement présent sur l'AT III dénaturée et non pas sur l'AT III native en solution.

On peut notamment utiliser comme anticorps connu l'anticorps DSM ACC 2167 pour trouver un anticorps complémentaire ; dans le cas où il est immobilisé, après sa réaction avec l'AT III ou l'AT III activée en solution, puis l'anticorps monoclonal à étudier, on pourra révéler avec un anticorps anti-IgG de souris marqué (sous réserve que l'anticorps immobilisé DSM ACC 2167 soit sous forme Fab ou F(ab')2 et que le conjugué anti-IgG de souris reconnaisse spécifiquement la partie Fc de l'anticorps à étudier) ; si c'est l'anticorps monoclonal à étudier qui est immobilisé, notamment par adsorption ou par l'intermédiaire d'un anticorps anti-IgG de souris, après sa réaction avec l'AT III ou l'AT III activée en solution, on pourra révéler avec l'anticorps DSM ACC 2167 marqué. Des procédés du même type peuvent être mis en oeuvre pour trouver des anticorps analogues de l'anticorps DSM ACC 2167, en utilisant un anticorps connu complémentaire.

Dans le cas où la sélection des hybridomes est faite avec un appareil d'étude de l'intensité des réactions immunologiques, tel que l'appareil BIACORE®, pour identifier un anticorps monoclonal reconnaissant mieux l'AT III activée en solution que l'AT III native, on fixera l'anticorps à étudier sur le biocapteur et on fera circuler dans la cellule soit une solution d'AT III activée, soit une solution d'AT III native.

Pour identifier un anticorps monoclonal reconnaissant l'AT III dénaturée sans reconnaître pratiquement l'AT III native ou activée, on fixera l'anticorps et on fera circuler successivement une solution d'AT III native, puis activée et enfin dénaturée par action de la guanidine.

Ce type d'étude est long et coûteux et on préfère présélectionner les hybridomes susceptibles de convenir en utilisant un procédé d'immunodosage.

Pour sélectionner les anticorps reconnaissant mieux l'AT III activée que l'AT III native, on pourra faire un dosage immunométrique de type sandwich, en phase solide.

Par exemple, les anticorps du sumageant à étudier seront immobilisés par adsorption sur une surface ou de préférence par l'intermédiaire d'un anticorps anti-immunoglobuline de souris, puis incubés avec une solution d'AT III contenant ou non l'oligosaccharide et après lavage, avec un anticorps anti-AT III marqué, avant d'effectuer la révélation de façon classique.

Pour sélectionner les anticorps reconnaissant l'AT III dénaturée on adsorbera l'AT III sur une surface éventuellement en présence de l'oligosaccharide avant incubation avec l'anticorps à étudier; la révélation sera effectuée avec un anticorps anti-immunoglobulines de souris, marqué.

Les anticorps sélectionnés répondant à l'invention peuvent être produits en quantité suffisante en ascite, sur des souris prétraitées au pristane ; ils peuvent alors être purifiés par chromatographie sur une colonne sur laquelle a été fixée de la protéine A, par exemple sur une colonne de sépharose® . Ces techniques sont bien connues et décrites pour les anticorps dirigés contre l'AT III dans Hybridoma, (1991), 10, p. 633-640.

Dans tous les immunoessais, les rapports molaires de l'oligosaccharide activateur et de l'AT III peuvent être supérieurs à ceux que l'on peut trouver in vivo ; néanmoins, la concentration d'activateur utilisée ne sera pas de préférence multipliée par plus de 10 par rapport à sa concentration habituelle, *in vivo,* tandis que la concentration en AT III restera proche de sa concentration physiologique, comprise entre 1 µM et 4 pM environ.

Selon un autre aspect, l'invention conceme un procédé de détermination in vitro de l'AT III activée par un glycosaminoglycane sulfaté, ou un oligosaccharide sulfaté, analogue biologique de l'héparine, qui consiste à effectuer un immunodosage, dit de type sandwich ou à deux sites, en utilisant un anticorps monoclonal qui reconnaît mieux l'AT III activée par un glycosaminoglycane sulfaté ou par un des oligosaccharides sulfatés, ayant une activité sur l'AT III analogue à celle de l'héparine, que l'AT III en solution et un anticorps monoclonal qui ne reconnaît pratiquement pas l'AT III native et l'AT III activée mais reconnaît l'AT III dénaturée par action de la guanidine ou de l'un de ses sels, ou par adsorption.

Les anticorps monoclonaux convenant pour ces dosages peuvent être obtenus avec des animaux immunisés contre l'AT III en présence d'un glycosaminoglycane sulfaté qui peut être différent de celui présent dans l'échantillon à déterminer et l'immunisation est, de préférence, en général réalisée avec un oligosaccharide sulfaté qui complexe l'AT III et a une activité antithrombotique, tel que le pentasaccharide A, précédemment cité. Néanmoins, notamment parce qu'après traitement d'un sujet par l'un quelconque des glycosaminoglycanes activateurs, seulement une faible proportion de l'AT III est sous forme activée, il est nécessaire de tracer la courbe d'étalonnage permettant de déterminer la quantité d'AT III sous forme activée dans un liquide biologique, avec des solutions d'AT III activée par le même glycosaminoglycane que celui connu que l'on veut étudier.

En utilisant les anticorps monoclonaux selon l'invention, il est possible d'effectuer des immunodosages de type sandwich (ou concentration - saturation). Selon ces types de dosages, un premier anticorps est immobilisé sur une surface solide. La solution à doser est ensuite déposée sur l'anticorps immobilisé. Celui ci immunocapture l'antigène qui dans le cas présent est l'antithrombine III humaine. Un deuxième anticorps couplé à un marqueur, tel que $I^{125}$, enzyme ou biotine est ensuite incubé. Après lavage, le complexe premier anticorps immobilisé - antigène - deuxième anticorps marqué est révélé.

Le dosage est dit "en un temps" lorsque le deuxième anticorps marqué est simultanément incubé avec l'échantillon contenant l'antigène à doser.

Le dosage est dit "en deux temps" lorsqu'on effectue d'abord l'incubation de l'échantillon contenant l'antigène à doser avec l'un des anticorps puis, après un éventuel lavage on introduit le deuxième anticorps et on incube à nouveau.

Selon une variante, l'immunodosage est effectué en un temps, c'est à dire que l'on incube la solution à étudier simultanément avec les deux anticorps, avant de séparer l'anticorps de marquage n'ayant pas réagi et d'effectuer la révélation par une technique appropriée.

Plus particulièrement selon ce procédé, l'anticorps qui reconnaît l'AT III dénaturée est préalablement immobilisé sur un support solide avant d'être mis en présence d'une solution contenant de l'AT III et d'une solution contenant un deuxième anticorps qui reconnaît mieux l'AT III activée par un glycosaminoglycane sulfaté ou l'un des oligosaccharides sulfatés, ayant une activité sur l'AT III analogue à celle de l'héparine, que l'AT III en solution, ledit deuxième anticorps étant conjugué à un marqueur enzymatique tel que la peroxydase.

Un procédé de détermination préféré consiste à effectuer une méthode sandwich en un temps, avec l'anticorps DSM ACC 2167 immobilisé et un anticorps complémentaire couplé à une enzyme, telle que la peroxydase, soit directement soit par l'intermédiaire du couple biotine/avidine, de façon connue en soi ; la révélation est effectuée, comme habituellement, par action de l'enzyme sur un substrat chromogène.

Selon une variante préférée de l'invention, l'immunodosage est effectué en deux temps. Dans ce cas, l'échantillon à doser sera d'abord incubé avec un anticorps immobilisé sur une surface solide. Après incubation et lavage, un deuxième anticorps couplé à une enzyme permettra de mettre en évidence l'immunocomplexe, anticorps immobilisé - antigène - deuxième anticorps marqué, qui sera formé après une deuxième incubation.

Un procédé de détermination particulièrement préféré consiste à effectuer un test sandwich en deux temps. Selon ce procédé un anticorps complémentaire à l'anticorps DSM ACC 2167 est immobilisé sur une surface solide, par

exemple dans un puits d'une microplaque. La solution à doser est ajoutée dans le puits. Après incubation et lavage, l'anticorps DSM ACC 2167 couplé à une enzyme, telle que la peroxydase, soit directement, soit par l'intermédiaire du couple biotine/anidine est rajouté. Après une deuxième incubation et lavage la révélation est effectuée par action de l'enzyme sur un substrat chromogène. On préfère que l'anticorps DSM ACC 2167 soit couplé directement à une enzyme.

On utilisera de préférence, comme solution diluante, un tampon phosphate salin, ou un tampon à base de trishydroxyméthylaminométhane (Tris), isotonique et dépourvu de calcium libre, étant donné les phénomènes d'association entre l'AT III et les glycosaminoglycanes. La solution sera rendue isotonique par exemple par addition de NaCl, en général à une concentration voisine de 0,15 M ; pour éliminer les ions calciques, on pourra les complexer par addition d'un agent séquestrant connu, tel que l'acide éthylènediaminotétraacétique (EDTA).

Toutefois, lors de la réalisation des tests sandwich en deux temps il est aussi possible d'utiliser des tampons contenant du $CaCl_2$.

L'immunodosage selon l'invention permet non seulement l'évaluation de l'activité anticoagulante due à l'antithrombine III activée par des glycosaminoglycanes sulfatés exogènes lors d'un traitement prophylactique ou thérapeutique, mais aussi celle de l'activité anticoagulante liée à l'AT III activée par les glycosaminoglycanes endogènes.

Le procédé immunologique selon l'invention trouve donc aussi son application dans l'évaluation des risques cardiovasculaires.

Dans ce qui suit on décrit des exemples de deux variantes du procédé de détermination de l'AT III dans le plasma de sujets traités avec une héparine de bas poids moléculaire, précédée de la description de l'obtention d'anticorps monoclonaux conformes à l'invention.

**EXEMPLE 1** - Obtention des anticorps monoclonaux

1. Immunisation des animaux

On administre par voie intrapéritonéale à quatre souris balb/c une dose journalière de 20 µg par souris du pentasaccharide A pendant 5 jours ainsi que le second jour par voie sous-cutanée, 20 µg d'AT III humaine en présence d'adjuvant complet de Freund. Un rappel selon le même protocole est effectué un mois plus tard et quelques jours après on sélectionne la souris dont le sérum donne la réponse immunologique la plus forte vis à vis de l'AT III en présence ou non du pentasaccharide A. La sélection des souris est effectuée par un immunoessai classique de type ELISA dans des cupules sur les parois desquelles a été fixé un anticorps anti-IgG de souris. Le sérum à tester y est mis à incuber puis après lavage, une solution d'AT III humaine à 1 µg/ml dans un tampon phosphate salin (PBS) contenant 0,1 % de Tween® 20 ou la même solution contenant en outre 1 µg/ml du pentasaccharide A sont introduits. Après incubation deux heures à température ambiante puis un lavage, on introduit une solution aqueuse contenant 0,5 µg/ml d'anticorps polyclonal anti-AT III, directement couplé à la peroxydase et après 30 minutes d'incubation, on révèle de façon classique.

La sélection des souris peut aussi être effectuée de la façon suivante avec l'AT III adsorbée : 100 µl de solution à 10 µg/ml d'AT III dans un tampon PBS contenant ou non 10 µg/ml de pentasaccharide A sont incubés dans les puits d'une plaque de microtitration convenable, pendant une nuit à +4°C ou 1 heure à 37°C, pour réaliser de façon connue l'adsorption de l'AT III sur les parois ; après lavage des puits, 100 µl de sérum dilué de 1000 à 10000 fois dans du tampon phosphate y sont incubés et après lavage, un anticorps anti-IgG murin conjugué à une enzyme est introduit, puis révélé de façon habituelle. La souris dont le sérum avait le meilleur titre a été choisie.

2. Hybridation lymphocytaire

La fusion est réalisée par action du polyéthylène-glycol, avec des cellules myélomateuses de souris, de la lignée P3-X63-Ag-8653 décrite dans J. Immunol. Methods, (1979), 123, p. 1548-1550, et des cellules spléniques de la souris sélectionnée. Les hybridomes sont cultivés sur les milieux habituels à base de RPMI, commercialisés par Gibco, contenant du sérum de veau foetal, de l'hypoxanthine, de la thymidine et de l'aminoptérine puis sélectionnés en appliquant les mêmes méthodes que pour la caractérisation des sérums de souris. Les hybridomes sélectionnés sont ensuite clonés par dilution limite à partir d'une dilution à 50 cellules par ml, à raison de 100 µl par puits d'une plaque de clonage.

3. Etude de l'affinité des anticorps pour l'AT III et l'AT III activée

On utilise un appareil BIACORE® dont le signal exprimé en unités de résonance, est une fonction linéaire de la quantité de protéines fixée sur la surface du biocapteur entre 1 pg/mm$^2$ et 30 ng/mm$^2$ pour des protéines de masse moléculaire comprise entre 25000 et 150000 environ. On immobilise sur la surface sensible par liaison covalente à l'hydrogel qui la recouvre, selon la méthode préconisée par le fabricant, un anticorps de lapin anti-immunoglobuline

de souris sur lequel les anticorps à étudier sont immunofixés à partir d'une solution à 100 µg/ml dans un tampon phosphate 10 mM contenant 150 mM de NaCl et 5 mM d'EDTA, pH 7,4 ; on note les signaux obtenus en faisant circuler du plasma humain normal dilué avec le tampon, de façon qu'il contienne environ 0,52 µM d'AT III et le même plasma dilué contenant l'un des glycosaminoglycanes sulfatés à une concentration de 52 µM.

Le tableau I donne les mesures relevées pour 12 surnageants d'hybridomes avec une héparine de bas poids moléculaire, la Fraxiparine ® (DCI Nadroparine calcique), classés en quatre groupes ; les anticorps du groupe 1 ne reconnaissent pratiquement pas l'AT III native ou activée en solution ; ceux du groupe 2 reconnaissent de la même façon l'AT III native et activée et ne répondent pas à l'invention ; les anticorps du groupe 3 reconnaissent mieux l'AT III native que l'AT III activée et ne répondent pas à l'invention, par contre les anticorps du groupe 4 reconnaissent mieux l'AT III activée par l'héparine de bas poids moléculaire et peuvent être utilisés pour effectuer le procédé de l'invention pour la détermination de l'AT III activée ; leurs anticorps complémentaires seront sélectionnés parmi ceux du Groupe I.

Tableau I

| ANTICORPS | SIGNAL | | GROUPE |
|---|---|---|---|
| | AT III | AT III activée* | |
| SR at 01 | 17 | 23 | 1 |
| SR at 02 | 67 | 60 | 1 |
| SR at 03 | 70 | 56 | 1 |
| SR at 04 | 115 | 120 | 2 |
| SR at 05 | 548 | 336 | 3 |
| SR at 06 | 465 | 260 | 3 |
| SR at 07 | 404 | 123 | 3 |
| SR at 08 | 367 | 207 | 3 |
| SR at 09 | 395 | 121 | 3 |
| SR at 10 | 382 | 123 | 3 |
| SR at 11 | 208 | 297 | 4 |
| SR at 12 | 346 | 421 | 4 |

* rapport molaire activateur/AT III = 100

On a aussi dans les mêmes conditions mesuré les signaux obtenus avec les anticorps SR at II et SR at 12 pour l'AT III en présence de différents glycosaminoglycanes sulfatés ; les résultats figurent dans le tableau II :

Tableau II

| ACTIVATEUR | SIGNAL (AT III activé*) Ac SR at 11 | SIGNAL (AT III) Ac SR at 12 |
|---|---|---|
| **Héparine** | 236 | 121 |
| **Fraxiparine** | 160 | 121 |
| **Pentasaccharide A** | 29 | 86 |

* rapport molaire activateur/AT III = 10

On constate qu'il serait préférable de ne pas utiliser l'anticorps SR at 11 pour une étude de l'activation in vivo de l'AT III par le pentasaccharide A tandis qu'il conviendrait lors d'une activation par l'héparine.
Lorsque l'on désire appliquer le procédé selon l'invention dans le cas d'un activateur quelconque, on pourra choisir l'anticorps SR at 12.

Les constantes d'association des anticorps monoclonaux du groupe 3 et 4 (SR at 07, SR at 11 et SR at 12) avec l'antithrombine III ont été aussi évaluées en présence et en absence d'une héparine de bas poids moléculaire (HBPM) et notamment de la nadroparine calcique.
Comme les résultats du Tableau III le démontrent, la constante d'association des deux anticorps monoclonaux du groupe 4 augmente en présence d'une héparine de bas poids moléculaire, contrairement à celle de l'anticorps SR at 07 du Groupe 3.

Pour les essais il a été utilisé un appareil BIACORE®, et les essais ont été effectués dans les conditions décrites précédemment. A noter que les essais en présence de la nadroparine calcique ont été réalisés en maintenant constant le rapport molaire héparine de bas poids moléculaire/AT III.

La concentration en AT III plasmatique a varié de 0,520 µM à 0,00325 µM.

Tableau III

| Groupe - Anticorps | | Plasma seul | | | Plasma avec HBPM | | |
|---|---|---|---|---|---|---|---|
| | | kon (1/Ms) | koff (1/s) | Ka (1M) | kon (1/Ms) | koff (1/s) | Ka (1M) |
| 3 | SR at 07 | 3,6E+05 | 5,4E-04 | 6,6E+08 | 2,2E+05 | 9,6E-04 | 2,3E+08 |
| 4 | SR at 11 | 2,0E+05 | 3,7E-03 | 5,3E+07 | 5,0E+05 | 1,6E-03 | 3,2E+08 |
| 4 | SR at 12 | 2,7E+04 | 4,7E-04 | 5,8E+07 | 3,5E+05 | 5,4E-04 | 6,4E+08 |

kon (1/Ms) = constante de vitesse d'association
koff (1/s) = constante de vitesse de dissociation
Ka (1/M) = contante d'affinité

4. Sélection d'un anticorps complémentaire à ceux du groupe 4

Cet anticorps est recherché parmi ceux qui donnent des signaux faibles au BIACORE®, et reconnaissent l'AT III dénaturé en solution.

De l'AT III purifiée, commerciale, est dénaturée par un traitement avec du chlorhydrate de guanidine : on laisse incuber 18 heures à +4°C une solution de tampon phosphate 10 mM contenant 50 µg/ml d'AT III, du NaCl 150 mM, de la guanidine 0,9 M, à pH 7,4 et laisse incuber 18 heures à +4°C ; la solution obtenue est dialysée contre le tampon phosphate 10 mM contenant du NaCl 150 mM, pH 7,4 pour obtenir une solution d'AT III dénaturée à 500 µg/ml.

La sélection est effectuée en utilisant les résultats d'un immunoessai en phase solide : les anticorps à tester sont adsorbés dans les puits d'une microplaque par contact pendant 18 heures à +4°C d'une solution d'anticorps à 10 µg/ml dans du tampon phosphate salin ; les puits sont lavés et 100 µl d'une solution d'AT III native ou dénaturée à 10 µg/ml dans un tampon phosphate 10 mM contenant du NaCl 150 mM, de l'EDTA 5 mM à pH 7,4 sont introduits ; après une heure d'incubation à température ambiante et lavage, on met dans les puits 0,05 µg d'un anticorps polyclonal anti AT III couplé à la peroxydase, en solution à 0,5 µg/ml. Après une heure d'incubation et des lavages, on révèle par addition de $H_2O_2$ et d'ortho-phénylènediamine puis arrêt de la réaction par addition de $H_2SO_4$.

Le rapport des absorptions de la lumière vers 490 nm pour l'essai avec l'AT III native par rapport à celui avec l'AT III dénaturée est inférieur à 0,1 pour l'anticorps SR at 02 alors qu'il est supérieur à 0,4 pour les anticorps SR at 01 et SR at 03 ; ces derniers ne font donc pas partie de l'invention. L'anticorps SR at 02 a été déposé à la collection DSM sous le ACC 2167

**EXEMPLE 2** - Obtention d'une courbe d'étalonnage pour le procédé d'immunodétermination de l'AT III activée par une héparine de bas poids moléculaire - Dosage en un temps.

On introduit dans les puits d'une plaque de microtitration 100 µl d'une solution à 10 µg/ml de l'anticorps SR at 02 en solution dans un tampon phosphate 10 mM, pH 7,4 contenant NaCl 150 mM et EDTA 5 mM. Après une incubation de 18 heures à +4°C et des lavages avec la solution tamponnée, on introduit 50 µl d'une solution à 0,5 mg/ml diluée au 1/40000 de l'anticorps SR at 12, conjugué à la peroxydase et 50 µl d'une solution de référence contenant du plasma normal et l'héparine de bas poids moléculaire (Fraxiparine) et on laisse incuber deux heures à température ambiante avant de laver et d'effectuer la révélation par addition d'un substrat chromogène de l'enzyme par exemple l'orthophényldiamine et de $H_2O_2$. L'absorption lumineuse est déterminée *à* 490 nm ou 492 nm, après addition d'$H_2SO_4$ 4N dans les puits.

La solution de référence est obtenue en diluant 10 fois dans un tampon phosphate la solution, résultant de l'incubation (par exemple 2 heures), à température ambiante, d'un mélange de 800 µl du tampon phosphate précédent avec 100 µl de plasma normal dont la concentration en AT III est estimée voisine de 2,6 µM, et 100 µl d'une solution de Fraxiparine contenant selon les cas de 0,26 µM à 260 µM d'activateur.

L'anticorps est conjugué à la peroxydase selon une méthode bien connue, qui consiste à faire réagir l'anticorps et la peroxydase activée par traitement au periodate de sodium avant de stabiliser le composé obtenu par traitement avec du borohydrure de sodium (méthode de Nakane).

La figure 1 donne en coordonnées semi-logarithmiques, en ordonnée l'absorbance à 490 nm et en abscisse les concentrations de l'activateur, exprimées en pM de Fraxiparine dans les 100 µl introduits dans le milieu.

**EXEMPLE 3** - Immunodosage dans le plasma d'un sujet traité par la Fraxiparine - Dosage en un temps

On administre à des volontaires sains par voie sous-cutanée, après un premier prélèvement de sang destiné à déterminer leur concentration plasmatique en AT III native, 35000 unités de Fraxiparine ; on leur prélève ensuite 1 ml de sang toutes les 1/2 heures pendant les 5 premières heures puis après 6, 8, 12, 18, 24, 30, 36 et 48 heures. On détermine sur chaque prélèvement la concentration du plasma en AT III activée par la méthode selon l'invention décrite dans l'exemple 2. Sur la figure n° 2 on a représenté l'absorbance mesurée en fonction du temps.

**EXEMPLE 4** - Immunodosage de l'AT III activée in vitro avant et après traitement à l'héparinase - Dosage en un temps

Une solution à 2,7 μM d'AT III purifiée dans un tampon phosphate 10 mM contenant NaCl 150 mM, EDTA 5 mM, de pH 7,4 est incubée (par exemple deux heures) à température ambiante, ainsi que la même solution contenant de l'héparine à la même concentration que l'AT III ; ces solutions sont ensuite diluées 100 fois dans le même tampon et incubées pendant une heure à température ambiante avec de l'héparinase ; cette réaction est effectuée en introduisant 0,5 ml de solution diluée dans un flacon de 1000 UI d'héparinase Hepzyme Dade commercialisé par Baxter sous la référence B 4240-10, (héparinase EC 4.2.2.7.)
On effectue l'immunodosage selon le procédé de l'invention déjà décrit à l'exemple 2 avec de l'anticorps SR at 02 immobilisé et l'anticorps SR at 12 couplé à la biotine par action de la N-hydroxy-succinimidobiotine, à la concentration de 1 μg/ml. La révélation est alors effectuée en ajoutant 50 μl d'une solution d'avidine couplée à la peroxydase, commercialisée par Pierce sous la référence 29994 et diluée 500 fois dans le tampon phosphate ; après 15 minutes d'incubation, les cupules de la plaque sont lavées 4 fois et l'activité de la peroxydase résiduelle est mesurée comme à l'exemple 2. La densité optique des deux échantillons sans héparine est très faible, voisine de 0,1 tandis que l'échantillon contenant de l'héparine a une densité optique voisine de 2, qui après traitement à l'héparinase n'est plus que de 0,5.

**EXEMPLE 5** - Obtention d'une courbe d'étalonnage pour le procédé d'immunodétermination de l'AT-III activée par une héparine non fractionnée - Dosage en deux temps

On introduit dans les puits d'une plaque de microtitration 100 μl d'une solution à 10 μg/ml d'anticorps SR at 12 en solution dans une solution tampon phosphate 10 mM (pH 7,4) contenant NaCl 150 mM et EDTA 5 mM. Après une incubation pendant 18 heures à +4°C et des lavages avec la solution tampon on introduit 100 μl de plasma surchargé ou non en héparine non fractionnée et préalablement dilué au 1/100 dans la solution tampon phosphate.
On incube 45 minutes à température ambiante et on lave avec la solution tampon phosphate. On ajoute ensuite 100 μl d'une solution à 0,5 mg/ml diluée au 1/2500 de l'anticorps SR at 02, conjugué à la peroxydase. Pour la dilution on utilise la même solution tampon phosphate décrite ci-dessus. On incube pendant 45 minutes et on lave avec la même solution tampon.
La révélation est effectuée par addition d'un substrat chromogène de l'enzyme, par exemple en ajoutant 100 μl d'une solution d'orthophénylènediamine et de $H_2O_2$. Après 15 minutes à température ambiante, la réaction est arrêtée par addition de 50 μl de $H_2SO_4$ 4 N et l'absorption est lue à 492 nm.
La solution de référence est obtenue en diluant au 1/100 avec du tampon phosphate, 100 μl de plasma normal dont la concentration en AT III est voisine de 2,6 pM.
Pour la gamme d'étalonnage le plasma a été surchargé avant dilution avec des quantités croissantes d'une héparine non fractionnée (HNF). Les rapports molaires HNF/AT III étaient de 0/1 (référence) à 1/1. La figure 3 donne en ordonnée l'absorbance à 492 nm et en abscisse les concentrations de l'activateur exprimées en moles de HNF/mole d'AT III. Le signal mesuré avec ce dosage en deux temps est tout à fait bien corrélé avec l'activité anti-Xa, évalué par un dosage biologique, (R = 0,980). Des comparaisons ont été réalisées avec du plasma provenant soit de volontaires sains soit a avec des malades traités par la spécialité Fraxiparine®.
Lors de ces essais, l'activité anti-Xa a été évaluée par la technique Rotachrome® de Stago.

**EXEMPLE 6** - Evaluation de l'activité anticoagulante plasmatique après occlusion veineuse - Mise en oeuvre du dosage en deux temps

Pour mettre en évidence une éventuelle augmentation du taux des glycosaminoglycanes endogènes circulants lors d'une lésion/stimulation endothliale comme celle réalisée avant et après occlusion veineuse, une étude a été menée chez 6 volontaires sains et 17 malades atteints de pathologies cardiovasculaires plus ou moins graves.
L'épreuve de stimulation de l'endothélium vasculaire est par exemple le test à la veinostase. Ce test consiste à placer sur un membre (en général un bras, plus rarement une jambe) d'un patient, un brassard ajusté à 100 mm de mercure. Après 10 minutes d'attente, le sang est récupéré. Un prélèvement avant veinostase est également réalisé.

Ce test induit une hémoconcentration (déterminée par l'hématocrite) ainsi qu'une certaine agression de l'endothélium vasculaire, provoquant la libération de substances présentes dans ou à la surface des cellules endothéliales. Ce test est habituellement utilisé pour apprécier l'activité fibrinolytique de ces cellules. Une injection de desmopressine provoque une stimulation pharmacologique de l'endothélium, avec des effets similaires à une veinostase.

Essai I

Afin d'éviter toute interférence due aux protéines plasmatiques, l'immunodosage a été effectué avec le plasma dilué au 1/1000 de la manière suivante :
Le plasma de 6 volontaires sains et de 17 malades est prélevé sur citrate et conservé à -20°C jusqu'au moment du dosage.
L'anticorps SR at 12 est adsorbé sur microplaque selon le protocole indiqué dans l'exemple 5. On introduit ensuite sur les microplaques, contenant de l'anticorps SR at 12, 100 µl de plasma dilué au 1/1000 dans un tampon phosphate 10 mM (pH = 7,4) contenant du NaCl 150 mM et EDTA 5 mM. On incube 45 minutes à température ambiante et on lave avec la solution tampon phosphate indiquée ci-dessus.
On ajoute ensuite 100 µl d'une solution de l'anticorps SR at 02 conjugué à la peroxydase (dilué au 1/500 dans la solution tampon phosphate). Après incubation pendant 45 minutes à température ambiante on procède au lavage des microplaques avec la même solution tampon.

L'activité peroxydasique résiduelle est révélée par addition d'un substrat chromogène et notamment de 100 µl d'une solution d'orthophénylènediamine et de $H_2O_2$. Après 15 minutes la réaction est arrêtée par addition de 50 µl de $H2SO_4$ 4N et l'absorbance est lue à 492 nm. Le signal mesuré dans les prélèvements après veinostase est corrigé en tenant compte de l'hémoconcentration.

Les premiers résultats obtenus lors de cette évaluation directe n'ont pas permis de mettre en évidence une différence significative entre les malades et les volontaires sains.

Par contre, il a été démontré que la différence des signaux (Δ) mesurée après et avant veinostase, patient par patient, selon la formule suivante :

$$\Delta = \text{(signal corrigé* mesuré après veinostase) - (signal mesuré avant veinostase)}$$

$$\text{* signal corrigé = (signal lu) x } \frac{\text{hématocrite avant veinostase}}{\text{hématocrite après veinostase}}$$

est significativement plus faible chez les malades ayant une pathologie cardiovasculaire que chez les volontaires sains. Dans les mêmes conditions expérimentales et avec d'autres marqueurs de l'hémostase [tels que l'antithrombine totale (AT totale), fragments 1+2 de la prothrombine (F1+2) ou complexe thrombine-antithrombine (complexe T-AT)], il n'existe pas de différence significative entre les volontaires sains et les malades. Le procédé d'immunodosage selon l'invention constitue un outil très performant pour détecter les risques cardiovasculaires.

Essai 2

Pour confirmer ces résultats et démontrer que l'augmentation du signal mesuré avec le plasma prélevé après veinostase est provoquée par un glycosaminoglycane endogène circulant, l'essai suivant a été réalisé. Les plasmas (avant et après veinostase), de trois volontaires sains ont été dosés avant et après traitement à l'héparinase selon le protocole suivant:

Les plasmas ont été dilués au 1/250 dans une solution tampon (pH 7,4) contenant du Tris 10 mM, NaCl 150 mM, $CaCl_2$ 5 mM et hirudine 5 µg/ml, 500 µl du plasma dilué ont été incubés dans un flacon d'Hepzyme pendant 2 heures à température ambiante. Les plasmas ont été ensuite dilués au 1/4 dans une solution tampon phosphate 10 mM (pH = 7,4) contenant NaCl 150 mM et EDTA 5 mM.
On procède ensuite au dosage immunologique comme indiqué à l'essai 1 ci-dessus.

Les résultats obtenus indiqués à la figure 4 démontrent qu'un traitement à l'héparinase des plasmas prélevés avant veinostase ne modifie pas significativement le signal. Par contre le traitement à l'héparinase des plasmas prélevés après veinostase diminue significativement le signal.

Le signal ainsi diminué est cependant supérieur à celui du plasma prélevé avant veinostase.

Les résultats des essais 1 et 2 laissent supposer qu'une anomalie endothéliale chez les sujets présentant des thromboses veineuses à répétition serait la cause d'une prédisposition au risque thrombotique.

Il semblerait que chez un individu normal, les cellules endothéliales, suite à une agression vasculaire, libéreraient dans le flux circulatoire une activité anticoagulante. Cette activité anticoagulante qui serait liée à l'AT III activée, associée aux glycosaminoglycanes endogènes (ou substances hépariniques) diminuerait chez les malades ayant une pathologie cardiovasculaire. Cette diminution serait due à la libération (plus faible chez les malades) des glycosaminoglycanes endogènes.

Les résultats de la figure 4 laisseraient aussi supposer que la substance libérée dans le sang au cours de la veinostase serait probablement un glycosaminoglycane du type "héparine" puisque le signal mesuré augmente très significativement après veinostase et qu'il est en partie diminué par un traitement à l'héparinase.

**Revendications**

1. Procédé de dosage de l'antithrombine III (AT III) activée par un glycosaminoglycane sulfaté ou un oligosaccharide sulfaté dont l'effet in vivo sur l'AT III est analogue à celui de l'héparine, par un immunodosage de type sandwich en phase solide, caractérisé en ce qu'on détermine à l'aide d'un anticorps spécifique d'un épitope exclusivement présent sur l'AT III dénaturée, les complexes immunologiques formés entre l'AT III activée et un anticorps spécifique de l'AT III activée.

2. Procédé selon la revendication 1 caractérisé en ce que les deux anticorps sont mis simultanément en présence de l'antigène.

3. Procédé selon les revendications 1 ou 2, dans lequel l'anticorps qui reconnaît l'AT III dénaturée est préalablement immobilisé sur une surface solide, avant d'être mis en présence d'une solution contenant de l'AT III et d'une solution contenant un deuxième anticorps qui reconnaît mieux l'AT III activée par un glycosaminoglycane sulfaté ou l'un des oligosaccharides sulfatés ayant une activité sur l'AT III analogue à celle de l'héparine, que l'AT III en solution, ledit deuxième anticorps étant conjugué à un marqueur enzymatique tel que la peroxydase.

4. Procédé selon la revendication 1, caractérisé en ce que l'échantillon contenant l'antigène à doser est d'abord incubé avec un anticorps spécifique de l'AT III activée, puis, après un éventuel lavage, le deuxième anticorps qui reconnaît l'AT III dénaturée est introduit et l'ensemble est incubé à nouveau.

5. Procédé selon les revendications 1 ou 4, dans lequel l'anticorps qui reconnaît mieux l'AT III activée par un glycosaminoglycane sulfaté ou l'un des oligosaccharides sulfatés ayant une activité sur l'AT III analogue à celle de l'héparine, que l'AT III en solution, est préalablement immobilisé sur une surface solide, avant d'être mis en présence d'une solution contenant de l'AT III.

6. Procédé selon les revendications 1, 4 et 5, caractérisé en ce que le deuxième anticorps qui est introduit après l'incubation du premier anticorps fixé sur un support solide avec l'échantillon contenant l'antigène à doser, est un anticorps qui reconnaît l'AT III dénaturé et il est couplé à une enzyme telle que la peroxydase.

7. Procédé selon l'une quelconque des revendications 1 à 6, caractérisé en ce qu'il est réalisé dans un milieu ne contenant pas d'ions calcium libres.

8. Procédé selon l'une quelconque des revendications 1 à 7, caractérisé en ce que le milieu comprend un complexant des ions calcium.

9. Procédé selon l'une quelconque des revendications 1 à 8, caractérisé en ce que ranticorps qui reconnaît l'AT III dénaturée est celui sécrété par l'hybridome déposé à la collection DSM sous le N° ACC 2167.

10. Procédé selon l'une quelconque des revendications 1 à 9, caractérisé en ce que les glycosaminoglycanes sulfatés et les oligosaccharides sulfatés sont ceux qui se fixent sur l'AT III comme l'héparine et en ce que les glycosaminoglycanes sulfatés sont choisis parmi l'héparine naturelle, et les héparines de bas poids moléculaire.

11. Procédé selon la revendication 10, caractérisé en ce que l'oligosaccharide est le pentasaccharide de formule :

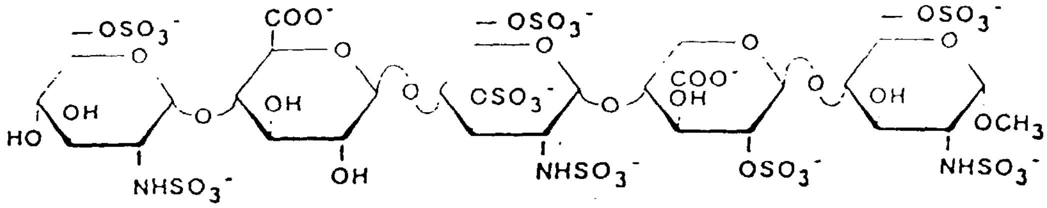

**12.** Anticorps monoclonal spécifique de l'AT III activée par un glycosaminoglycane sulfaté, dont l'effet in vivo sur l'AT III est analogue à celui de l'héparine, ledit anticorps, sous forme complexée avec l'AT III activée favorisant l'apparition d'un épitope exclusivement présent sur l'AT III dénaturée.

**13.** Anticorps monodonal selon la revendication 12, caractérisé en ce que le glycosaminoglycane sulfaté est le pentasaccharide de formule :

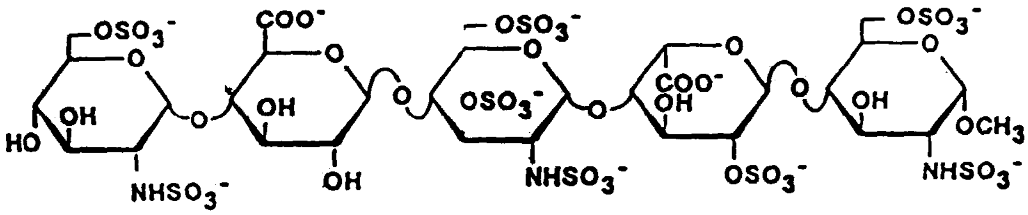

**14.** Anticorps monoclonal dirigé contre un épitope exclusivement présent sur l'AT III dénaturée, qui reconnaît spécifiquement les complexes immunologiques formés entre l'AT III activée par un glycosaminoglycane sulfaté ou un oligosaccharide sulfaté dont l'effet in vivo sur l'AT III est analogue à celui de l'héparine et un anticorps monoclonal selon la revendication 12 ou 13.

**15.** Anticorps monoclonal selon la revendication 14 sécrété par l'hybridome déposé à la collection DSM sous le N° ACC 2167.

**16.** Procédé d'obtention d'un anticorps monoclonal selon l'une des revendications 12 à 15, caractérisé en ce qu'on immunise les animaux avec l'antithrombine III humaine et un oligosaccharide sulfaté dont l'effet in vivo sur l'AT III est analogue à celui de l'héparine et que l'on sélectionne les hybridomes qui sécrètent des anticorps présentant les propriétés immunologiques recherchées, c'est-à-dire ceux qui ont plus d'affinité pour l'AT III activée en solution que pour l'AT III native et ceux qui reconnaissent spécifiquement l'AT III dénaturée, par exemple par la guanidine et ses sels, notamment le chlorhydrate de guanidine et ne reconnaissent pratiquement pas l'AT III native et l'AT III activée.

**17.** Procédé selon la revendication 16, caractérisé en ce que l'oligosaccharide sulfaté est le pentasaccharide de formule :

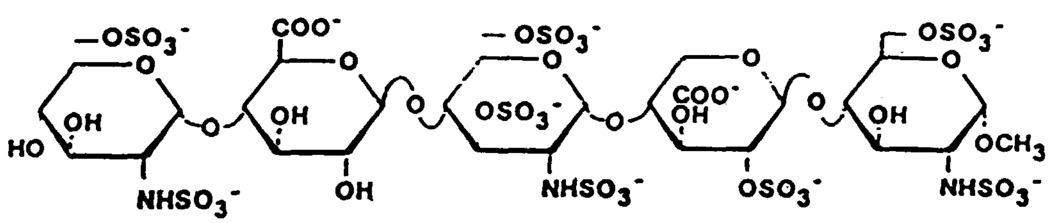

**18.** Procédé selon l'une des revendications 16 ou 17, pour l'isolement d'anticorps selon les revendications 12 à 15, caractérisé en ce que la sélection des hybridomes convenables est effectuée à l'aide d'un appareil de mesure des affinités immunologiques.

**19.** Procédé selon l'une des revendications 16 ou 17 caractérisé en ce que la sélection est effectuée avec un appareil

qui détermine la vitesse de fixation de macromolécules sur une surface en mesurant la réflexion d'un rayon lumineux polarisé et mettant en jeu le phénomène de résonance plasmonique de surface.

**20.** Procédé selon l'une quelconque des revendications 16 à 19 caractérisé en ce qu'une présélection des hybridomes sécrétant dos anticorps selon les revendications 12 à 15 est effectuée avant leur clonage par dilution limite, par un immunoessai avec un anticorps polyclonal anti-AT III.

**21.** Utilisation du procédé selon l'une quelconque des revendications 1 à 11 pour détecter les risques cardiovasculaires.

**Patentansprüche**

**1.** Verfahren zur Bestimmung von Antithrombin III (AT III), das durch ein sulfatiertes Glycosaminoglycan oder ein sulfatiertes Oligosaccharid aktiviert ist, dessen Effekt in vivo auf AT III analog dem von Heparin ist, durch eine Immunobestimmung vom Sandwich-Typ in fester Phase, dadurch charakterisiert, daß man mit Hilfe eines für ein ausschließlich auf denaturiertem AT III vorhandenes Epitop spezifischen Antikörpers die immunologischen Komplexe bestimmt, die sich zwischen aktiviertem AT III und einem für das aktivierte AT III spezifischen Antikörper bilden.

**2.** Verfahren nach Anspruch 1, dadurch charakterisiert, daß die beiden Antikörper gleichzeitig mit dem Antigen zusammengebracht werden.

**3.** Verfahren nach den Ansprüchen 1 oder 2, wobei der Antikörper, der denaturiertes AT III erkennt, zuvor auf einer festen Oberfläche immobolisiert wird, bevor er mit einer AT III enthaltenden Lösung und mit einer Lösung zusammengebracht wird, die einen zweiten Antikörper enthält, der durch ein sulfatiertes Glycosaminoglycan oder eines der sulfatierten Oligosaccharide, die eine Aktivität auf AT III analog der von Heparin aufweisen, aktiviertes AT III besser erkennt als AT III in Lösung, wobei der zweite Antikörper mit einem enzymatischen Marker wie Peroxidase konjugiert ist.

**4.** Verfahren nach Anspruch 1, dadurch charakterisiert, daß die Probe, die das zu bestimmende Antigen enthält, zuvor mit einem für aktiviertes AT III spezifischen Antikörper inkubiert wird, danach nach einer möglichen Waschung der zweite Antikörper, der denaturiertes AT III enkennt, eingeführt wird und alles von neuem inkubiert wird.

**5.** Verfahren nach den Ansprüchen 1 oder 4, wobei der Antikörper, der durch ein sulfatiertes Glycosaminoglycan oder eines der sulfatierten Oligosaccharide, die eine Aktivität auf AT III analog der von Heparin aufweisen, aktiviertes AT III besser erkennt als AT III in Lösung, zuvor auf einer festen Oberfläche immobilisiert wird, bevor er mit einer AT III enthaltenden Lösung zusammengebracht wird.

**6.** Verfahren nach den Ansprüchen 1, 4 und 5, dadurch charakterisiert, daß der zweite Antikörper, der nach Inkubation des ersten auf einen festen Träger fixierten Antikörpers mit der das zu messende Antigen enthaltenden Probe eingeführt wird, ein Antikörper ist, der denaturiertes AT III erkennt und an ein Enzym wie Peroxidase gekoppelt ist.

**7.** Verfahren nach einem der Ansprüche 1 bis 6, dadurch charakterisiert, daß es in einem Medium durchgeführt wird, das keine freien Calciumionen enthält.

**8.** Verfahren nach einem der Ansprüche 1 bis 7, dadurch charakterisiert, daß das Medium ein Komplexierungsmittel für Calciumionen enthält.

**9.** Verfahren nach einem der Ansprüche 1 bis 8, dadurch charakterisiert, daß der Antikörper, der denaturiertes AT III erkennt, derjenige ist, der vom Hybridom sezerniert wird, das bei der Sammlung DSM unter der Nr. ACC 2167 hinterlegt wurde.

**10.** Verfahren nach einem der Ansprüche 1 bis 9, dadurch charakterisiert, daß die sulfatierten Glycosaminoglycane und die sulfatierten Oligosaccharide diejenigen sind, die sich auf AT III wie Heparin fixieren, und dadurch, daß die sulfatierten Glycosaminoglycane zwischen natürlichem Heparin und Heparinen mit niedrigem Molekulargewicht ausgewählt werden.

**11.** Verfahren nach Anspruch 10, dadurch charakterisiert, daß Oligosaccharid das Pentasaccharid der Formel

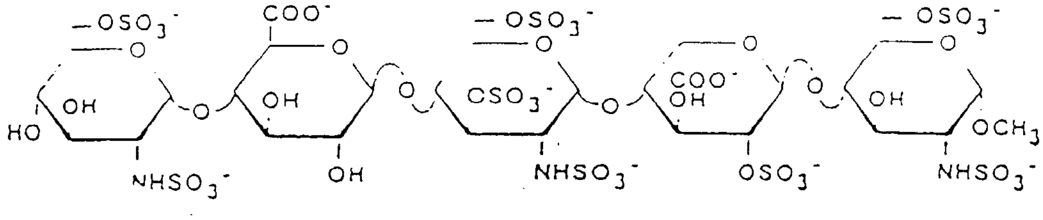

ist.

**12.** Monoklonaler Antikörper, der spezifisch ist für AT III, das durch ein sulfatiertes Glycosaminoglycan aktiviert ist, dessen Wirkung in vivo auf AT III analog der von Heparin ist, wobei der Antikörper in komplexierter Form mit aktiviertem AT III die Bildung eines Epitops begünstigt, das ausschließlich auf denaturiertem AT III vorhanden ist.

**13.** Monoklonaler Antikörper nach Anspruch 12, dadurch charakterisiert, daß das sulfatierte Glycosaminoglycan das Pentasaccharid der Formel ist:

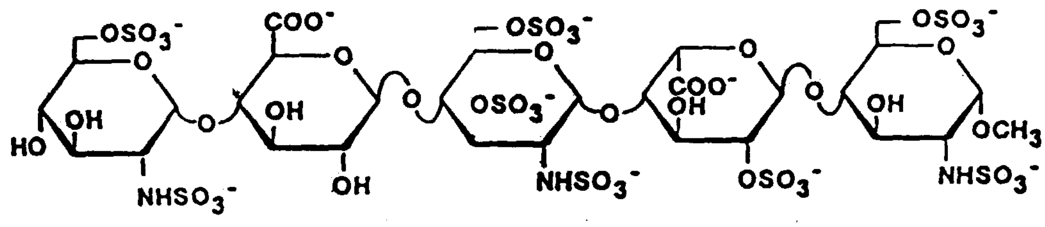

**14.** Monoklonaler Antikörper, der gegen ein Epitop gerichtet ist, das ausschließlich auf denaturiertem AT III vorhanden ist, der spezifisch die immonologischen Komplexe erkennt, die sich zwischen durch ein sulfatiertes Glycosaminoglycan oder ein sulfatiertes Oligosaccharid, dessen Wirkung in vivo auf AT III analog der von Heparin ist, aktiviertem AT III und einem monoklonalen Antikörper nach Anspruch 12 oder 13 bilden.

**15.** Monoklonaler Antikörper nach Anspruch 14, der durch das Hybridom sezerniert wird, das bei der Sammlung DSM unter der Nr. ACC 2167 hinterlegt wurde.

**16.** Verfahren zur Herstellung eines monoklonalen Antikörpers nach einem der Ansprüche 12 bis 15, dadurch charakterisiert, daß man die Tiere mit Human-Antithrombin III und einem sulfatiertem Oligosaccharid immunisiert, dessen Wirkung in vivo auf AT III analog ist der von Heparin, und daß man die Hybridome selektiert, die Antikörper sezernieren, die die gewünschen immonologischen Eigenschaften zeigen, d.h. solche, die mehr Affinität für aktiviertes AT III in Lösung als für natives AT III zeigen, und solche, die spezifisch denaturiertes AT III erkennen, beispielsweise durch Guanidin und seine Salze, insbesondere das Hydrochlorid von Guanidin, und die natives AT III und aktiviertes AT III praktisch nicht erkennen.

**17.** Verfahren nach Anspruch 16, dadurch charakterisiert, daß das sulfatierte Oligosaccharid das Pentasaccharid der Formel ist:

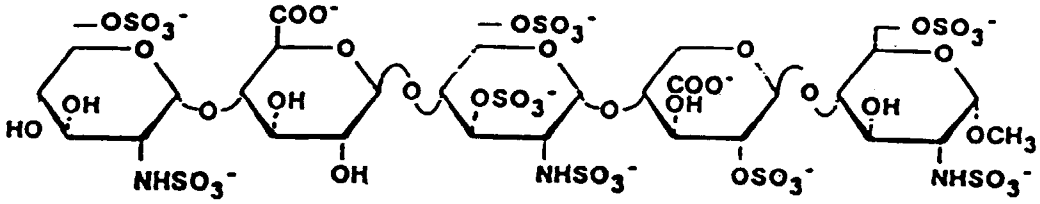

**18.** Verfahren nach einem der Ansprüche 16 oder 17 zur Isolierung des Antikörpers aus den Ansprüchen 12 bis 15, dadurch charakterisiert, daß die Selektion der gewünschten Hybridome mit Hilfe eines Meßapparates für die immonologischem Affinitäten durchgeführt wird.

**19.** Verfahren nach einem der Ansprüche 16 oder 17, dadurch charakterisiert, daß die Selektion mit einem Apparat durchgeführt wird, der die Fixierungsgeschwindigkeit von Makromolekülen auf einer Oberfläche durch Messung der Reflexion eines polarisierten Lichtstrahls bestimmt, und in dem das Phänomen der plasmonischen Oberflächenresonanz ausgenutzt wird.

**20.** Verfahren nach einem der Ansprüche 16 bis 19, dadurch charakterisiert, daß eine Vorselektion der Hybridome, die die Antikörper nach den Ansprüchen 12 bis 15 sezernieren, vor ihrer Klonierung durch Grenzverdünnung durch ein Immunoassay mit einem polyklonalen Antikörper Anti-AT III durchgeführt wird.

**21.** Verwendung des Verfahrens nach einem der Ansprüche 1 bis 11 zum Nachweis von kardiovaskulären Risiken.

**Claims**

**1.** Process for determining antithromhin III (AT III) activated by a sulphated glycosaminoglycan or a sulphated oligosaccharide of which the in vivo effect on AT III is analogous to that of heparin, by solid phase sandwich-type immunoassay, characterised in that the immunological complexes formed between activated AT III and an antibody specific to activated AT III are determined by means of an antibody specific to an epitope exclusively present on denatured AT III.

**2.** Process according to claim 1, characterised in that the two antibodies are placed in the presence of the antigen simultaneously.

**3.** Process according to claim 1 or 2, wherein the antibody recognising denatured AT III is previously immobilised on a solid surface before being placed in the presence of a solution containing AT III and a solution containing a second antibody that recognises AT III activated by a sulphated glycosaminoglycan or one of the sulphated oligosaccharides having an activity on AT III analogous to that of heparin better than it does AT III in solution, the second antibody being conjugated with an enzyme marker, such as peroxidase.

**4.** Process according to claim 1, characterized in that the sample containing the antigen to be determined is first of all incubated with an antibody specific to activated AT III, then, after optional washing, the second antibody recognising denatured AT III is introduced and the whole is incubated again.

**5.** Process according to claim 1 or 4, wherein the antibody that recognises AT III activated by a sulphated glycosaminoglycan or onc of the sulphated oligosaccharides having an activity on AT III analogous to that of heparin better than it does AT III in solution, is previously immobilised on a solid surface before being placed in the presence of a solution containing AT III.

**6.** Process according to claims 1, 4 and 5, characterised in that the second antibody which is introduced after incubation of the first antibody fixed on a solid support with the sample containing the antigen to be determined is an antibody recognising denatured AT III and it is coupled to an enzyme, such as peroxidase.

**7.** Process according to any one of claims 1 to 6, characterised in that it is carried out in a medium that does not contain free calcium ions.

**8.** Process according to any one of claims 1 to 7, characterised in that the medium comprises an agent complexing calcium ions.

**9.** Process according to any one of claims 1 to 8, characterised in that the antibody recognising denatured AT III is that secreted by the hybridoma deposited at the DSM collection under No. ACC 2167.

**10.** Process according to any ONe of claims 1 to 9, characterised in that Lhe sulphated glycosaminoglycans and the sulphated oligosaccharides are those which attach themselves to AT III, such as heparin, and in that the sulphated glycosaminoglycans are selected from natural heparin and low-molecular-weight heparins.

**11.** Process according to claim 10, characterised in that the oligosaccharide is the pentasaccharide of the formula:

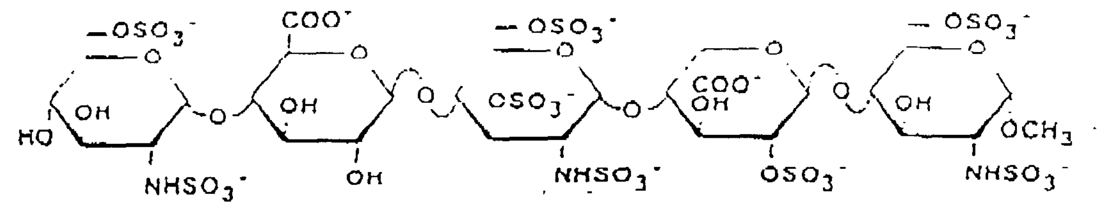

**12.** Monoclonal antibody specific to AT III activated by a sulphated glycosaminoglycan of which the in vivo effect on AT III is analogous to that of heparin, the antibody, in a form complexed with activated AT III, promoting the appearance of an epitope exclusively present on denatured AT III.

**13.** Monoclonal antibody according to claim 12, characterised in that the sulphated glycosaminoglycan is the pentasaccharide of the formula:

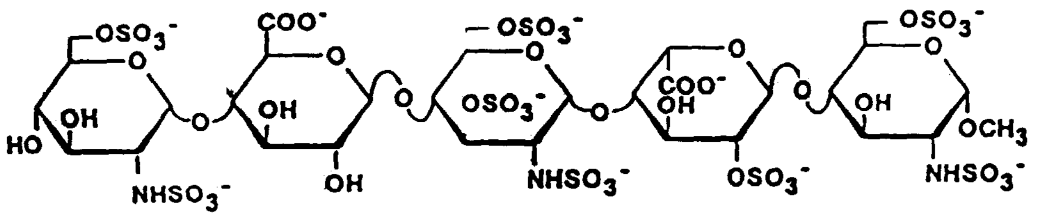

**14.** Monoclonal antibody directed against an epitope exclusively present on denatured AT III, that specifically recognises the immunological complexes formed between AT III activated by a sulphated glycosaminoglycan or a sulphated oligosaccharide of which the in vivo effect on AT III is analogous to that of heparin and a monoclonal antibody according to claim 12 or 13.

**15.** Monoclonal antibody according to claim 14, secreted by the hybridoma deposited at the DSM collection under No. ACC 2167.

**16.** Process for obtaining a monoclonal antibody according to any one of claims 12 to 15, characterised in that animals are immunised with human antithrombin III and a sulphated oligosaccharide of which the in vivo effect on AT III is analogous to that of heparin and in that the hybridomas that secrete antibodies having he desired immunological properties are selected, that is to say, those which have more affinity for activated AT III in solution than for native AT III and those which specifically recognise AT III that is denatured, for example, by guanidine and its salts, especially guanidine hydrochloride, and which exhibit practically no recognition of native AT III and activated AT III.

**17.** Process according to claim 16, characterised in that the sulphated oligosaccharide is the pantasaccharide of the formula:

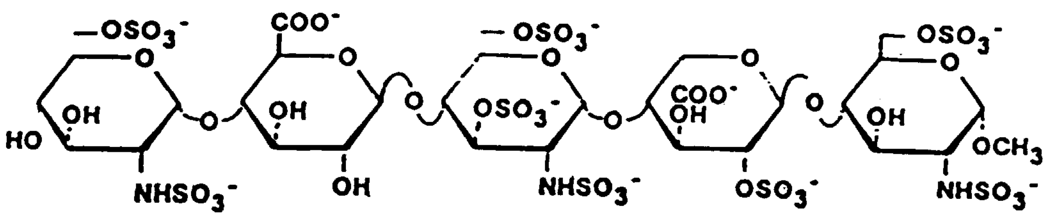

**18.** Process according to either claim 16 or claim 17, for the isolation of antibodies according to claims 12 to 15, characterised in that the selection of the appropriate hybridomas is carried out using an apparatus for measuring immunological affinity.

**19.** Process according to either claim 16 or claim 17, characterised in that the selection is carried out with an apparatus that determines the rate of attachment of macromolecules to a surface by measuring the reflection of a polarised light beam and bringing into play the phenomenon of surface plasmonic resonance.

**20.** Process according to any one of claims 16 to 19, characterised in that a preselection of the hybridomas secreting antibodies according to claims 12 to 15 is carried out before they are cloned by limit dilution, by an immunoassay with an anti-AT III polyclonal antibody.

**21.** Use of the process according to any one of claims 1 to 11 for detecting cardiovascular risk.

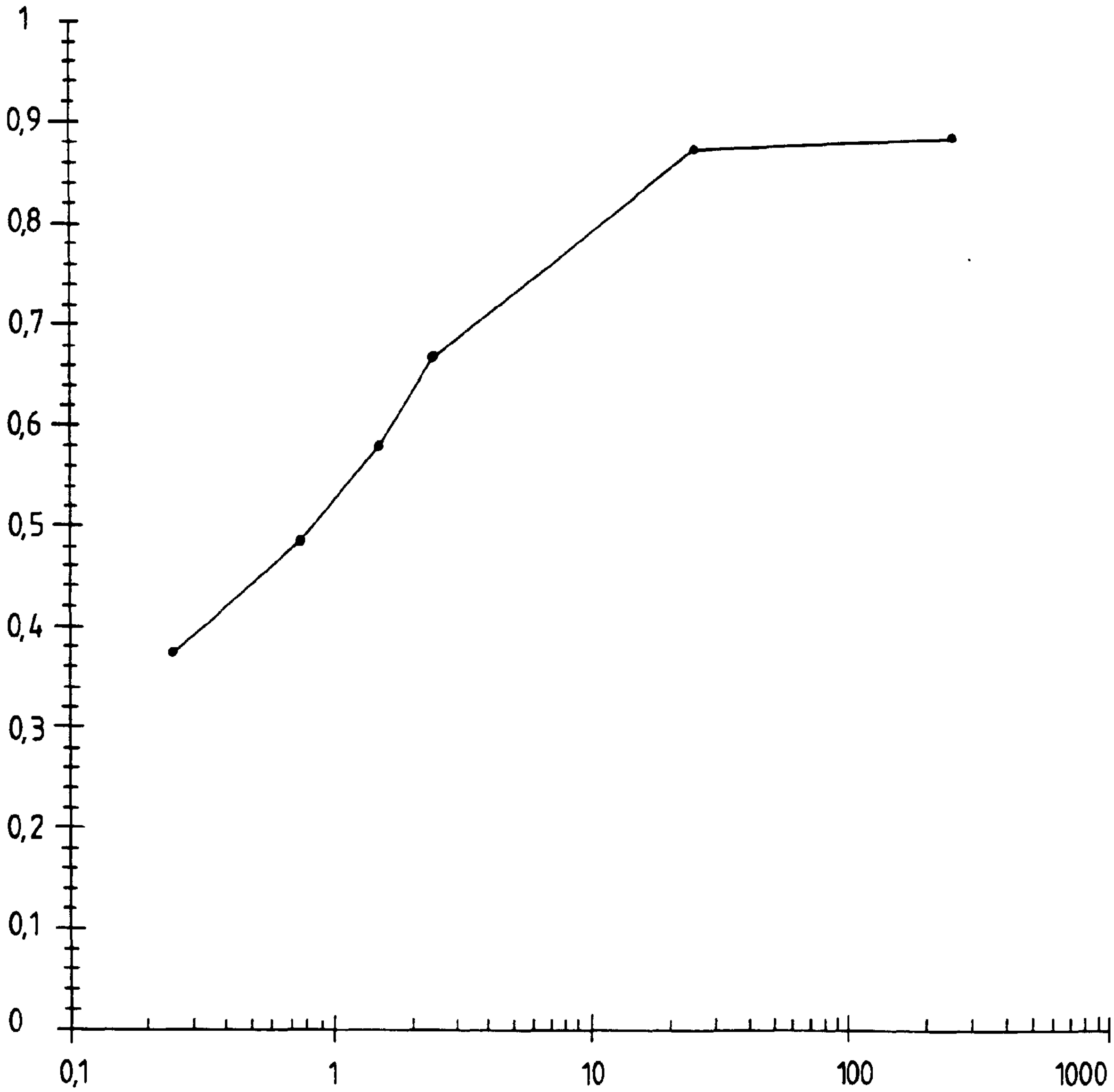


FIG.1

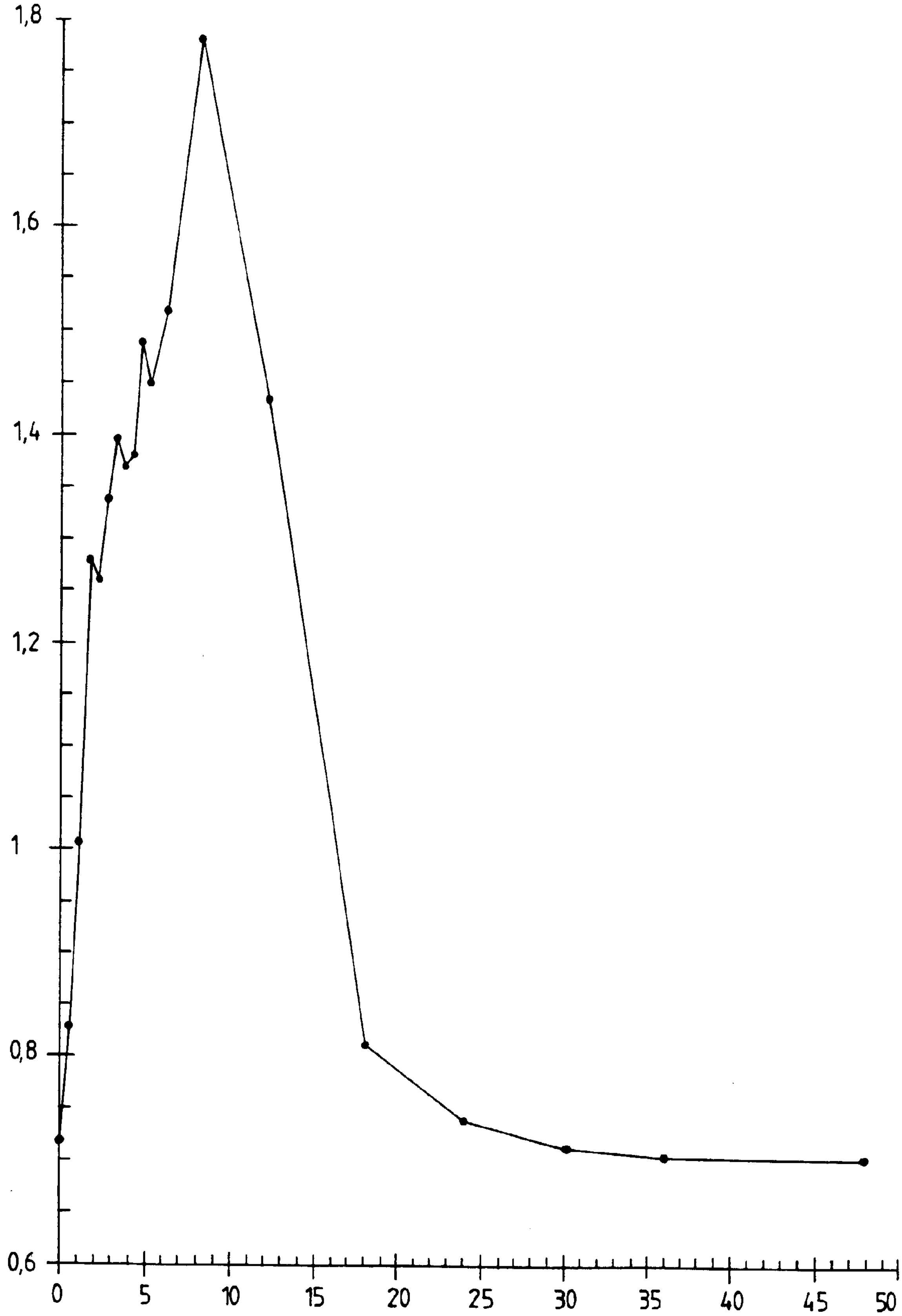


FIG. 2

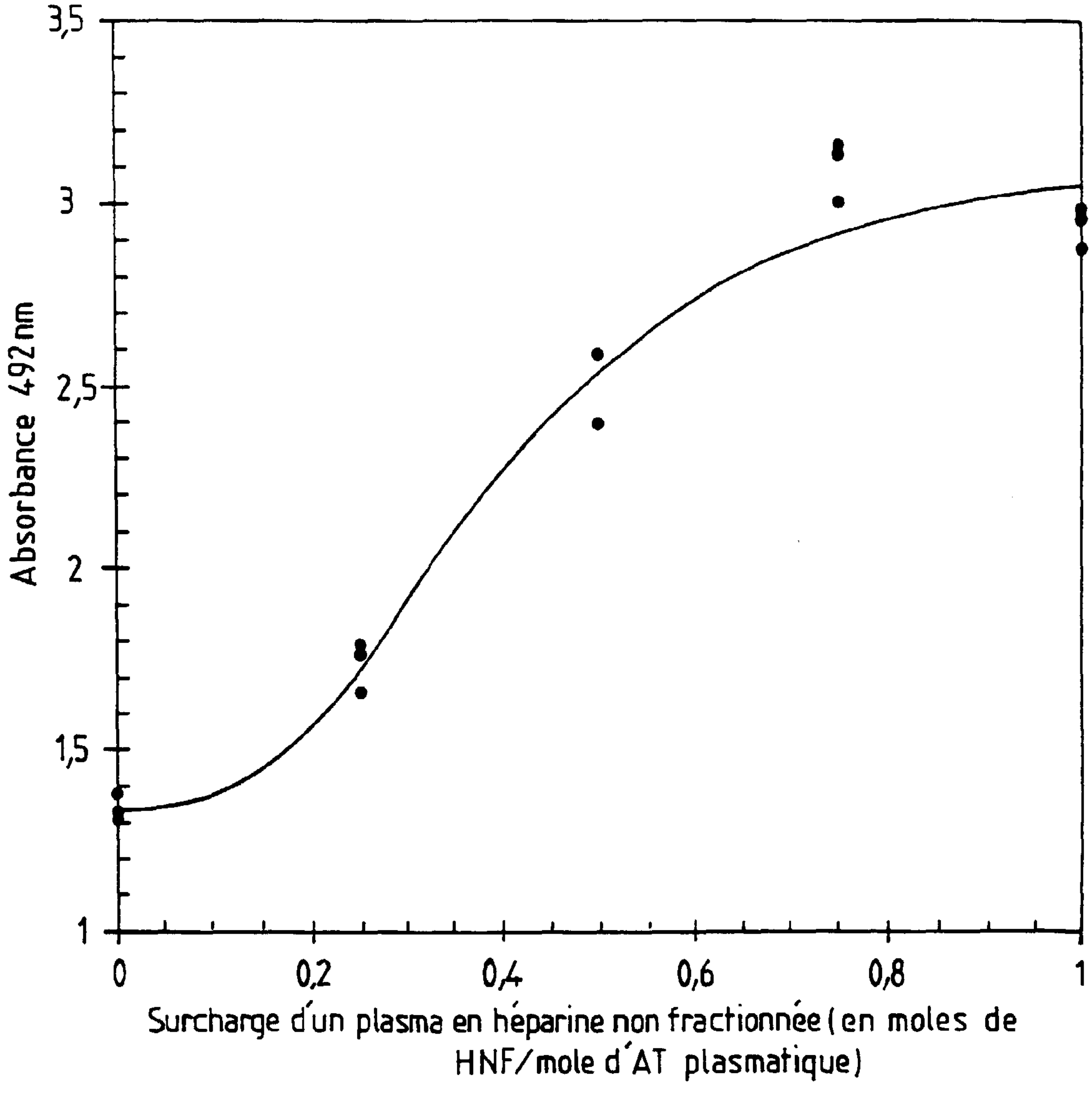
3,5
3
2,5
2
1,5
1
Absorbance 492 nm
0
0,2
0,4
0,6
0,8
1
Surcharge d'un plasma en héparine non fractionnée (en moles de HNF/mole d'AT plasmatique)

FIG. 3

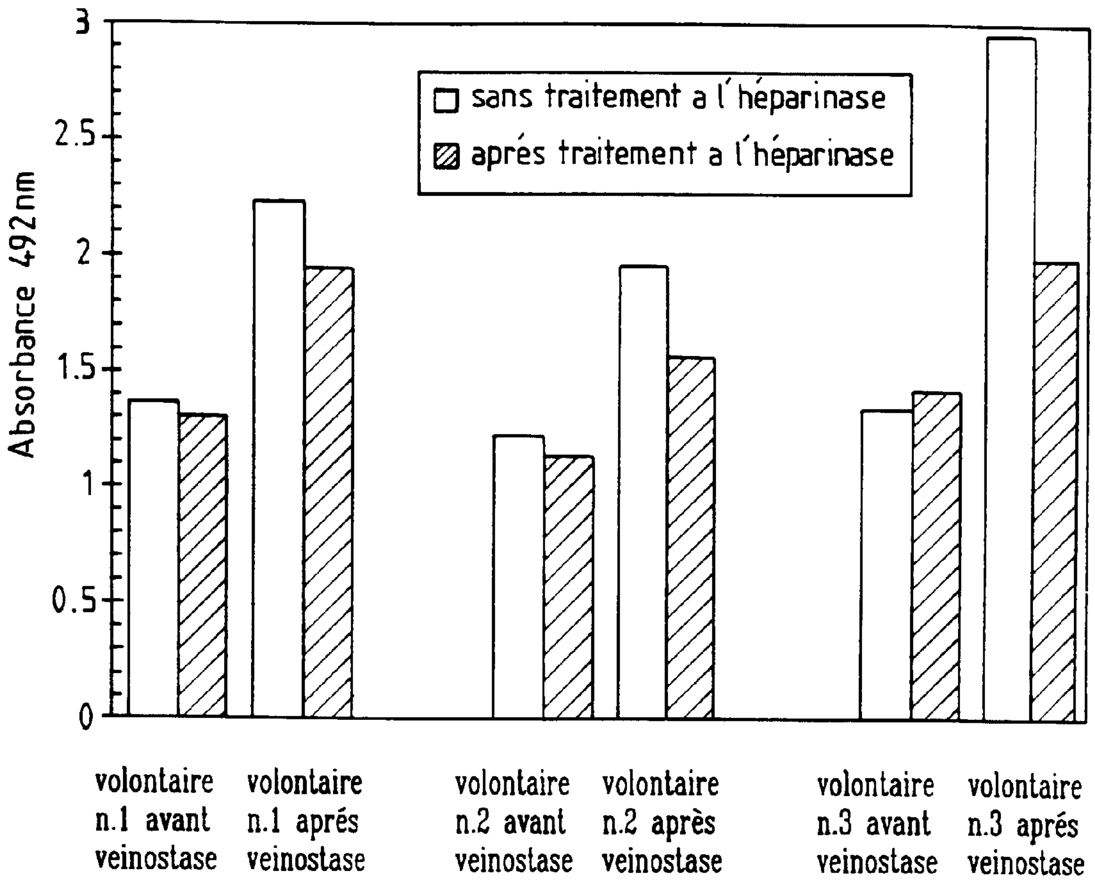
3
2.5
2
1.5
1
0.5
0
Absorbance 492 nm
sans traitement a l'héparinase
aprés traitement a l'héparinase
volontaire n.1 avant veinostase
volontaire n.1 aprés veinostase
volontaire n.2 avant veinostase
volontaire n.2 après veinostase
volontaire n.3 avant veinostase
volontaire n.3 aprés veinostase

FIG.4